# EUROPEAN PATENT SPECIFICATION

(11) **EP 3 902 516 B1**
(45) Date of publication and mention of the grant of the patent: **22.07.2026**
(21) Application number: 19832973.2
(22) Date of filing: 26.12.2019
(51) Int. Cl.: A61K 8/46, A61K 8/49, A61K 8/73, A61K 8/81, A61Q 17/04

(54) **COSMETIC COMPOSITION COMPRISING HYDROSOLUBLE UV FILTERS**
KOSMETISCHE ZUSAMMENSETZUNG ENTHALTEND WASSERLÖSLICHE UV
COMPOSITION COSMETIQUE COMPRENANT DES FILTRES UV HYDROSOLUBLES

(30) Priority: 26.12.2018 FR 1874156
(43) Date of publication of application: 03.11.2021
(73) Proprietor: L'OREAL, 75008 Paris (FR)
(72) Inventor: ROUDOT, Angélina, 94152 CHEVILLY LA RUE (FR); ADER, Laure, 94152 CHEVILLY LA RUE (FR); GILLANT, Flavie, 94152 CHEVILLY LA RUE (FR); DOUEZAN, Stephane, 94152 CHEVILLY LA RUE (FR)
(74) Representative: Lavoix
(86) International application number: PCT/EP2019/087050
(87) International publication number: WO 2020/136226

(56) References cited:
- EP-A1- 1 627 621
- EP-A1- 2 921 157
- US-A1- 2015 174 046
- DATABASE GNPD [online] MINTEL; 2 May 2011 (2011-05-02), OMBRELLE CANADA: "Complete SPF 30 Lotion", XP002794278, Database accession no. 1530058
- DATABASE GNPD [online] MINTEL; 1 June 2015 (2015-06-01), LA ROCHE POSAY: "Mist SPF 50", XP002794279, Database accession no. 3215907
- DATABASE GNPD [online] MINTEL; 3 August 2015 (2015-08-03), LA ROCHE POSAY: "Sunscreen Lotion SPF 50+", XP002794280, Database accession no. 3308557
- DATABASE GNPD [online] MINTEL; 1 May 2008 (2008-05-01), LESCOJA CORPOTATION: "Complete Head Care Lotion", XP002794281, Database accession no. 913899

## Description

This invention relates to new compositions, and particularly cosmetics comprising at least one hydrosoluble filter capable of absorbing UV from 320 to 400 nm (UVA), and at least one hydrosoluble filter capable of absorbing UV from 280 to 320 nm (UVB), in a pharmaceutically acceptable medium; and at least one specific thickening agent.

It is known that radiation with wavelengths between 280 nm and 400 nm enables tanning of the human epidermis, while radiation with wavelengths between 280 and 320 nm, known as UVB rays, impede the development of a natural tan. Exposure can also lead to a prejudicial change in the biomechanical properties of the epidermis, resulting in the appearance of wrinkles leading to premature aging of the skin.

It is also known that UVA rays with a wavelength of between 320 and 400 nm penetrate into the skin into the skin more deeply than UVB rays. UVA rays cause immediate and persistent tanning of the skin. Daily exposure to UVA rays, even short duration, under normal conditions, can damage collagen and elastin fibers, which results in a modification to the microrelief of the skin, the appearance of wrinkles and unequal pigmentation (spots and uneven color).

A wide variety of photoprotective compositions to protect keratin materials and particularly the skin, against the harmful effects induced by UVA and/or UVB radiation, are already known in prior art. Essentially, they contain a combination of several organic or inorganic UV filters, transferred in the oily phase and/or the aqueous phase as an anti-UV active agent and are generally offered in emulsion or gel type galenical form.

It is also known that high filter ratios are necessary to reach high level filter efficiencies.

However, high ratios with UV filters do not facilitate the production of compositions that have a stable and pleasing texture.

Thus, formulations with a high filter capacity generally have uncomfortable or even unpleasant sensorial aspects concealing the freshness and comfort of formulas. In particular, the weak point of photoprotective formulations with a high protection index is often a significantly greasy sensation, and therefore lack of lightweight feeling of the textures obtained, but also a white appearance in application and therefore not invisible on the skin.

Furthermore, the introduction of UV filters with a high content usually causes destabilization problems. This instability can even sometimes cause a phase change in the emulsion and/or a loss of viscosity of the composition, making the formulation inefficient or even unusable.

Aqueous dosages have already been considered to mitigate the above-mentioned undesirable effects, and in particular to obtain a fresh effect on application while being invisible on the skin. However, these aqueous compositions containing UV filters are generally sticky and therefore uncomfortable.

Consequently, there is a need for an aqueous photoprotective composition with a high level of UV protection that is perfectly stable and homogeneous, in other words that is unaffected by demixing phenomena.

There is also a need for an aqueous photoprotective composition with a high level of UV protection and that is also transparent. There is also a need for a photoprotective composition that is transparent even after application on the skin.

There is also a need for an aqueous photoprotective composition with a high level of UV protection, without a greasy or sticky finish on the skin, and with a pleasant sensation, materialized particularly by easy sliding during application.

The purpose of this invention is to offer new cosmetic compositions providing a solution to these problems.

In particular, the purpose of this invention is to offer new aqueous compositions, particularly cosmetic, comprising hydrosoluble UV filters, that are stable and homogeneous.

The present invention thus relates to a composition, particularly a cosmetic composition, according to claim 1.

"Physiologically acceptable medium" means a medium compatible with keratin materials. "Keratin materials" refers to the skin and/or lips and/or hair.

Due to the presence of the thickening agent according to the invention, compositions according to the invention can be used to make a wide variety of textures, i.e. fluid to very viscous solutions.

These compositions are stable and homogeneous. "Homogeneous" means that the composition is in the form of a single phase, without grains visible to the naked eye in suspension in the composition or on the walls of the receptacle, and/or has a uniform texture, i.e. without any gel portions in solution.

Preferably, the composition according to this invention is transparent or translucent. According to some preferred embodiments, the composition according to the invention is a transparent composition.

The term transparent or translucent composition as used in the context of this invention means a composition with a turbidity value of less than 1000 NTU, preferably less than 800 NTU, preferably less than 500 NTU, preferably less than 200 NTU, preferably less than 150 NTU, and preferably less than 100 NTU. Preferably, the turbidity of compositions is equal to at least 1 NTU.

NTUs (nephelometric turbidity units) are units for measurement of the turbidity of a composition. The turbidity measurement is made, for example, with a model 2100P turbidity meter made by the Hach Company, the tubes used for the measurement being references AR397A cat 24347-06. The measurements are made at ambient temperature (from 20°C to 25°C).

Preferably, the composition is transparent and has a turbidity value equal to between 1 and 200 NTU, preferably between 1 and 150 NTU, and preferably less than 100 NTU.

Preferably, the composition is transparent after application on the skin, i.e. there is no white mark after spreading the composition on the skin.

Another purpose of this invention is a non-therapeutic method of cosmetic treatment of keratin fibers, preferably the skin, comprising application of a composition according to the invention on said keratin fibers.

### Hydrosoluble filters

The composition according to the invention comprises at least one hydrosoluble filter capable of absorbing UVA and at least one hydrosoluble filter capable of absorbing UVB.

The term "hydrosoluble UV filter" refers to any inorganic or organic filter that can be completely dissolved in molecular form in an aqueous liquid phase, or be dissolved in colloidal form (for example in micellar form) in an aqueous liquid phase.

### Hydrosoluble filters capable of absorbing UV from 320 to 400 nm (UVA)

Among hydrosoluble filters capable of absorbing UVA, mention may be made of:
- Terephthalylidene Dicamphor Sulfonic Acid made by CHIMEX under the name "MEXORYL SX";
- bis-benzoazolyl derivatives as described in EP 669 323, and US 2 463 264 and more specifically the Disodium Phenyl Dibenzimidazo Tetra-sulfonate compound sold under the trade name "NEO HELIOPAN AP" by Haarmann and REIMER.

Preferably, the hydrosoluble capable of absorbing UVA is terephthalylidene dicamphor sulfonic acid.

### Hydrosoluble filters capable of absorbing UV from 280 to 320 nm (UVB)

Among hydrosoluble filters capable of absorbing UVB, mention may be made of:
*derivatives of p-aminobenzoic acid (PABA), such as:*
PABA,
Glyceryl PABA, and
PEG-25 PABA sold under the name "UVINUL P25" by BASF.
Phenylbenzimidazole Sulfonic Acid particularly sold particularly under the trade name "EUSOLEX 232" by MERCK,
Ferulic acid,
Salicylic acid,
Diethanolamine (DEA) methoxycinnamate,
Benzylidene Camphor Sulfonic Acid manufactured under the name "MEXORYL SL" by CHIMEX, and
Camphor Benzalkonium Methosulfate manufactured under the name "MEXORYL SO" by CHIMEX.

The hydrosoluble filter capable of absorbing UVB is Phenylbenzimidazole Sulfonic acid.

### Combined hydrosoluble UVA and UVB filters

The composition according to the disclosure comprises at least one mixed hydrosoluble filter capable of absorbing UVA and UVB, such as a derivative of benzophenone comprising at least one sulfonic radical, such as particularly:
Benzophenone-4 sold under the trade name "UVINUL MS 40" by BASF,
Benzophenone-5, and
Benzophenone-9.

Preferably, the composition according to the invention comprises a total quantity of hydrosoluble filters equal to between 0.2% and 40% by weight, preferably between 0.5% and 40% by weight relative to the total weight of composition, preferably between 1% and 30% by weight, and preferably between 3% and 20% by weight.

The hydrosoluble filter capable of absorbing UVA is present in a quantity equal to between 0.2% and 40% by weight, preferably between 0.5% and 30% by weight relative to the total weight of composition, preferably between 1% and 20% by weight, and preferably between 1.5% and 10% by weight.

The hydrosoluble filter capable of absorbing UVB is present in a quantity equal to between 0.2% and 40% by weight, preferably between 0.5% and 30% by weight relative to the total weight of composition, preferably between 1% and 20% by weight, and preferably between 1.5% and 10% by weight.

When the hydrosoluble UV filter is of the sulfonic acid type, it is preferably associated with an organic base such as an alkanolamine.

The term "alkanolamine" means a C2-C10 compound comprising at least one primary, secondary or tertiary amine function, and at least one alcohol function, general primary. Among appropriate alkanolamines, mention may be made of tromethamine and triethanolamine.

### Thickening agent

The composition according to the invention comprises at least one thickening agent chosen from among synthetic anionic thickening polymers, non-ionic thickening polysaccharides, sulfated thickening polysaccharides and branched carboxylated thickening polysaccharides. The thickening agent according to the invention is polymeric.

"Carboxylated" means at least one carboxylic acid function.

In the framework of this application, a thickening agent is an agent capable of increasing by at least 10 times, preferably at least 15 times, and preferably at least 20 times, the viscosity of a mixture comprising 1% by weight of said agent, 0.9% by weight of triethanolamine and water at a temperature of 25°C.

Preferably, the composition according to the invention is substantially free (i.e. contains less than 0.4%, preferably less than 0.3%, preferably less than 0.2%, preferably less than 0.1% by weight relative to the total weight of the composition), and preferably is completely free, of any thickening agent chosen from among clays, mica, modified starches such as phosphate starches and gellan gum.

### Synthetic anionic thickening polymers

The composition according to the invention can comprise a synthetic anionic thickening polymer.

Preferably, the synthetic anionic thickening polymer is non-aromatic; this means that it does not contain any aromatic monomer.

Among synthetic anionic thickening polymers that can be used, mention can be made of cross-linked homopolymers or copolymers of acrylic or methacrylic acid, homopolymers of 2-acrylamido-2-methyl-propane sulfonic acid, cross-linked or not, and salts thereof, and copolymers of acrylamido-2-methyl propane sulfonic acid or its salts and of one or several non-ionic monomers, cross-linked or not, alone or in mixtures.

A first suitable family of thickening polymers is represented by cross-linked homopolymers of acrylic acid. Among homopolymers of this type, mention can be made of those cross-linked by an allyl alcohol ether in the sugar series, for example such as those sold under the names CARBOPOL 980, 981, 954, 2984 and 5984 by NOVEON or products sold under the names SYNTHALEN M and SYNTHALEN K by 3 VSA.

Such acrylic homopolymers can be present in the composition in particulate or non-particulate form. When they are present in particulate form, their average size in the hydrated state is preferably less than or equal to 10 µm, and even more preferably less than or equal to 5 µm. Their average size in the dry or non-hydrated state is preferably less than or equal to 2 µm, and more preferably less than or equal to 1 µm.

Preferably, the acrylic acid homopolymer is present in non-particulate form.

Preferably, an acrylic acid homopolymer that is at least partially neutralized is used. The homopolymer used in this invention is chosen particularly among sodium polyacrylates and potassium polyacrylates. Sodium polyacrylate is preferably used.

Acrylic polymers that are neutralized before their use include for example:
- sodium polyacrylates such as those marketed under the trade name Cosmedia SP^{®} containing 90% of dry material and 10% of water, or Cosmedia SPL^{®} in inverse emulsion containing about 60% of dry active material, an oil (hydrogenated polydecene) and a surfactant (PPG-5 Laureth-5), both sold by the Cognis company;
- partially neutralized sodium polyacrylates, particularly in the form of an inverse emulsion comprising at least one polar oil, for example that sold under the trade name Luvigel^{®} EM by the BASF company; and
- mixtures thereof.

An acrylic acid homopolymer that has not been previously neutralized can also be used, that is then partially or completely neutralized by any appropriate means before use and particularly by the addition of any base such as soda, potash, an alkanolamine such as triethanolamine. This results particularly in sodium polyacrylates. Potassium polyacrylates are also suitable for this invention.

A second suitable family of thickening polymers is represented by cross-linked copolymers of (meth)acrylic acid and C1-C30 alkyl acrylate.

These copolymers are cross-linked copolymers of (meth)acrylic acid and C1-C6 alkyl acrylate.

The monomer of (meth)acrylic acid is present preferably in quantities ranging from 20 to 80% by weight, and more particularly from 25 to 70% by weight, even more particularly from 35 to 60% by weight relative to the total weight of the copolymer.

The monomer of C1-C6 alkyl acrylate is present preferably in quantities ranging from 15 to 80% by weight and more particularly from 25 to 75% by weight and even more particularly from 40 to 65% by weight relative to the total weight of the copolymer. Among these monomers, mention can be made of methyl acrylate, ethyl acrylate, n-butyl acrylate, 2-hydroxyethyl acrylate, styrene, acrylamide, N,N-dimethylacrylamide, tertio-butylacrylamide, methyl methacrylate, ethyl methacrylate, n-butyl methacrylate, 2-hydroxyethyl methacrylate.

Such a copolymer is typically partially or entirely cross-linked by at least one conventional cross-linking agent. The cross-linking agents are in particular polyunsaturated compounds. These compounds are in particular polyalkenylethers of sucrose or of polyols, diallylphthalates, divinylbenzene, allyl (meth)acrylate, ethyleneglycol di(meth)acrylate, methylene bis-acrylamide, trimethylol propane tri(meth)acrylate, diallyl itaconate, diallyl fumarate, diallyl maleate, zinc (meth)acrylate, derivatives of castor oil or of polyols manufactured from unsaturated carboxylic acids. As a cross-linking agent, unsaturated monomer compounds and which comprise a reactive group able to react with an unsaturation in order to form a cross-linked copolymer can also be used. The content of cross-linking agent varies in general from 0.01 to 5% by weight and preferably from 0.03 to 3% by weight and even more particularly from 0.05 to 1% by weight relative to the total weight of the copolymer.

The preferred cross-linked copolymer of (meth)acrylic acid and C1-C6 alkyl acrylate according to the invention is chosen from among a cross-linked copolymer of methacrylic acid and C1-C6 alkyl acrylate, a cross-linked copolymer of acrylic acid and C1-C6 alkyl acrylate and more particularly a cross-linked copolymer of methacrylic acid and ethyl acrylate.

According to a particularly preferred embodiment, the copolymer of the invention may be in dispersion in water. The average size as a number of particles of copolymer in the dispersion is generally between 10 and 500 nm and preferably between 20 and 200 nm and more preferably between 50 and 150 nm.

Among the cross-linked copolymers of (meth)acrylic acid and C1-C6 alkyl acrylate, mention can be made of the product sold under the trade name VISCOATEX 538C by COATEX which is a cross-linked copolymer of (meth)acrylic acid and ethyl acrylate in C1-C4 in an aqueous dispersion at 38% of active material, or the product sold under the trade name ACULYN 33 by ROHM & HAAS which is a cross-linked copolymer of acrylic acid and ethyl acrylate in aqueous dispersion at 28% of active material. Mention can be made more particularly of the cross-linked methacrylic acid/ethyl acrylate copolymer in the form of an aqueous dispersion at 30% manufactured and sold under the name CARBOPOL AQUA SF-1 by NOVEON.

A third family of thickening agents is represented by homopolymers of 2-acrylamido-2-methyl-propane sulfonic acid (AMPS^{®}), cross-linked or not, or salts thereof. These homopolymers may or may not be cross-linked.

Such a homopolymer can have an average molecular weight number varying from 1,000 to 20,000,000 g/mole, preferably varying from 20,000 to 5,000,000 and even more preferably from 100,000 to 1,500,000 g/mole.

More particularly, 2-acrylamido-2-methylpropane-sulfonic acid and its partially or totally neutralized forms are used.

When the polymers are cross-linked, the cross-linking agents may be chosen from among olefinic polyunsaturation compounds routinely used for cross-linking polymers obtained by radical polymerization. As examples of cross-linking agents, mention may be made for example of divinylbenzene, diallylic ether, dipropyleneglycol-diallylether, polyglycol-diallylethers, triethyleneglycol-divinylether, hydroquinone-diallyl-ether, di(meth)acrylate of ethyleneglycol or tetraethyleneglycol, trimethylol propane triacrylate, methylene-bis-acrylamide, methylene-bis-methacrylamide, triallylamine, triallylcyanurate, diallylmaleate, tetraallylethylenediamine, tetra-allyloxy-ethane, trimethylolpropane-diallylether, allyl (meth)acrylate, allyl alcohol ether in the sugar series, or other allyl- or vinyl-ethers of polyfunctional alcohols, and allyl esters of phosphoric and/or vinylphosphonic acid derivatives, or mixtures of these compounds.

According to one preferred embodiment of the disclosure, the cross-linking agent is chosen from methylene-bis-acrylamide, allyl methacrylate or trimethylol propane triacrylate (TMPTA). The degree of cross-linking generally ranges from 0.01% to 10% in moles and more particularly from 0.2% to 2% in moles relative to the polymer.

The homopolymer only comprises monomers with a sulfonic group and, if it is cross-linked, one or several cross-linking agents.

Preferred homopolymers of 2-acrylamido-2-methylpropane-sulfonic acid are generally characterized by the fact that they comprise the following, randomly distributed:
a) from 90 to 99.9% by weight of patterns with the following general formula (1): 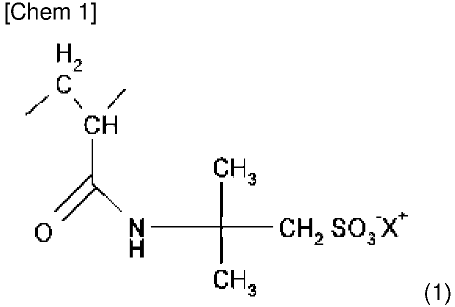 wherein X⁺ designates a proton, a alkaline metal cation, a alkaline-earth cation or the ammonium ion, not more than 10% mol of X⁺ cations that can be H⁺ protons;
b) from 0.01 to 10% by weight of cross-linking patterns originating from at least one monomer having a least two olefinic double bonds, proportions by weight being defined relative to the total weight of the polymer.

The more particularly preferred homopolymers according to the disclosure comprise 98 to 99.5% by weight of patterns with formula (1) and 0.2 to 2% by weight of cross-linking patterns.

As polymers of this type, mention may be made in particular of the cross-linked and neutralized homopolymer of 2-acrylamido 2-methylpropane sulfonic acid, marketed by Clariant under the tradename "Hostacerin AMPS^{®}" (CTFA name: ammonium polyacryldimethyltauramide).

The polymer can also be an amphiphilic homopolymer (or modified hydrophobic homopolymer) chosen from among statistical amphiphilic polymers of 2-acrylamido-2-methylpropane-sulfonic acid modified by reaction with an n-monoalkylamine or a C6-C22 di-n-alkylamine, such as those described in document WO-A-00/31154, that are grafted homopolymers.

A fourth family of suitable thickening agents is represented by copolymers of acrylamido-2-methyl-propane sulfonic acid, or salts thereof, and one or several non-ionic monomers. They may or may not be cross-linked.

When the polymers are cross-linked, the cross-linking agents may be chosen from among olefinic polyunsaturation compounds routinely used for cross-linking polymers obtained by radical polymerization. Such agents are described above.

According to one preferred embodiment of the invention, the cross-linking agent is chosen from methylene-bis-acrylamide, allyl methacrylate or trimethylol propane triacrylate (TMPTA). The degree of cross-linking generally ranges from 0.01% to 10% in moles and more particularly from 0.2% to 2% in moles relative to the polymer.

Copolymers according to the invention are obtained from AMPS^{®} and from one or a plurality of non-ionic hydrophilic or hydrophobic monomers with ethylene unsaturation and, if they are cross-linked, one or a plurality of cross-linking agents such as those defined above.

The monomer of 2-acrylamido-2-methylpropane sulfonic acid of the copolymer contained in the composition according to the invention is in free form or is partially or completely neutralized by an inorganic base (soda, potash, ammonia) or an organic base such as mono-, di-, or tri-ethanolamine, an aminomethylpropanediol, N-methyl-glucamine, basic amino acids such as arginine and lysine and a mix of these compounds.

Preferably, the monomer of 2-acrylamido-2-methylpropane sulfonic acid is partially or completely salified in the form of an ammonium or sodium salt.

Preferably, the monomer of 2-acrylamido-2-methylpropane sulfonic acid is completely salified, preferably in the form of an ammonium or sodium salt.

Copolymers of AMPS^{®} contain one or several non-ionic monomers chosen from among hydrosoluble monomers with ethylenic unsaturation, hydrophobic monomers or mixtures thereof.

Among non-ionic hydrosoluble monomers, mention may be made for example of:
- (meth)acrylamide,
- N-vinylacetamide and N-methyl N-vinylacetamide,
- N-vinylformamide and N-methyl N-vinylformamide,
- maleic anhydride,
- vinylamine,
- N-vinyllactames comprising a cyclic alkyl group with 4 to 9 carbon atoms such as N-vinylpyrrolidone, N-butyrolactame and N-vinylcaprolactame,
- vinyl alcohol with formula CH₂=CHOH,
- hydrosoluble vinylic monomers with the following formula (2): 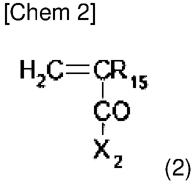 wherein:
   - R₁₅ is chosen from among H, -CH₃, -C₂H₅ or -C₃H₇
   - X₂ is chosen from among -OR₁₆ type alkyl oxides wherein R₁₆ is a linear or branched, saturated or unsaturated hydrocarbon radical, with 1 to 6 carbon atoms, possibly substituted by a halogen atom (iodine, bromine, chlorine, fluorine); a hydroxy group (-OH); ether.

For example, mention may be made of glycidyl (meth)acrylate, hydroxyethyl (meth)acrylate, and ethylene glycol, diethyleneglycol or polyalkyleneglycol (meth)acrylates.

Preferably, the hydrosoluble monomer is chosen from among acrylamide, vinylpyrrolidone, hydroxyalkyl(meth)acrylates, and more particularly vinylpyrrolidone.

Among copolymers of AMPS^{®} conforming with the invention with hydrophilic monomers, mention may for example be made of:
- copolymers of acrylamido-2-methyl propane sulfonic acid and vinylpyrrolidone, particularly such as the commercial product ARISTOFLEX AVC sold by CLARIANT,
- cross-linked acrylamide/acrylamido-2-methyl propane sodium sulfonate copolymers such as those used in the commercial product SEPIGEL 305 (INCI name: Polyacrylamide/C₁₃-C₁₄ Isoparaffin/Laureth-7) or that used in the commercial product sold under the name SIMULGEL 600^{®} (INCI name: Acrylamide / Sodium Acryloyldimethyltaurate / Isohexadecane/ Polysorbate-80) by SEPPIC;
- copolymers of AMPS^{®} and hydroxyethyl acrylate, such as for example sodium AMPS^{®}/hydroxyethyl acrylate copolymer like that used in the commercial product sold under the name SIMULGEL NS^{®} by SEPPIC (INCI name: Hydroxyethyl acrylate/Sodium Acryloyldimethyltaurate copolymer (and) Squalane (and) Polysorbate 60);
- hydrophobically modified copolymers of AMPS^{®} such as the copolymer known under the INCI name: AMMONIUM ACRYLOYLDIMETHYLTAURATE/STEARETH-25 METHACRYLATE CROSSPOLYMER marketed under the ARISTOFLEX HMS tradename by CLARIANT.

The concentration of synthetic anionic thickening polymer (i.e. active material), generally ranges from 0.05 to 35% by weight relative to the total weight of the composition, and preferably from 0.1 to 20% by weight, preferably from 0.2 to 10% by weight, and preferably from 0.25 to 5% by weight, and even more particularly from 0.3 to 3% by weight.

### Thickening polysaccharides

The composition according to the invention also comprises a thickening polymer chosen from among non-ionic thickening polysaccharides, sulfated thickening polysaccharides and branched carboxylated thickening polysaccharides.

Among non-ionic thickening polysaccharides, mention may be made in particular of non-ionic cellulosic thickening polysaccharides.

Celluloses that can be used in compositions according to this invention are chosen from among celluloses that do not contain a hydrophobic chain and non-ionic celluloses comprising one or several hydrophobic chains.

Celluloses used according to the invention are cellulose ethers. These celluloses are non-ionic hydroxyalkylcelluloses and particularly hydroxyethylcelluloses or hydroxypropylcelluloses. They may or may not contain a fatty chain. Among non-ionic celluloses that do not comprise a fatty chain, mention may be made of hydroxyethylcelluloses, hydroxypropylcelluloses and hydroxypropylmethylcelluloses. One particularly suitable hydroxyethylcellulose is Cellosize HEC QP-4400 H marketed by Amerchol (INCI name HYDROXYETHYLCELLULOSE).

Celluloses modified by groups comprising one or several non-ionic fatty chains that can be used according to this invention are in particular:
- non-ionic hydroxyethylcelluloses modified by groups comprising at least one fatty chain such as alkyl, arylalkyl, alkylaryl groups or mixtures thereof, and in which the alkyl groups are preferably in C8-C22, such as the NATROSOL PLUS GRADE 330 CS^{®} product (alkyls in C16) sold by AQUALON corresponding to the INCI name "CETYLHYDROXYETHYLCELLULOSE", or the BERMOCOLL EHM 100^{®} product sold by BEROL NOBEL,
- those modified by polyalkylene glycol alkyl phenol ether groups such as the AMERCELL POLYMER HM-1500 product (polyethylene glycol (15) nonyl phenol ether) sold by the AMERCHOL Company corresponding to the INCI name "NONOXYNYL HYDROXYETHYLCELLULOSE".

Among sulfated thickening polysaccharides, mention may be made in particular of sulfated polysaccharides without rhamnose. These sulfated polysaccharides are carrageenans.

Carrageenans are polysaccharides that make up the cell walls of various red algae (Rhodophycea) belonging to the Gigartinaceae, Hypneaceae, Furcellariaceae and Polyideaceae families. They comprise long anionic polyelectrolyte galactan chains. Their molecular mass can be more than 10⁶. These linear polymers, formed by disaccharide patterns, are composed of two D-galactopyranose units bonded alternately by α- and β-bonds. They are highly sulfated polysaccharides (20-50%) and the α-D-galactopyranosyl residues can be in 3',6'-anhydro form.

Initially, carrageenans were subdivided into two families depending on their solubility in potassium chloride (KCI). The fractions soluble in KCI were designated by "Kappa" prefixes while the "Lambda" terms were reserved for insoluble fractions. Later on, the classifications were based on the number and position of sulfate groups and the presence of the 3',6'-anhydro bridge on β-D-galactopyranosyl residues. This led to the four main families: κ, λ, β and ω.

The different types of carrageenans do not exist in the pure state, but in the form of hybrids. Thus in the natural state, κ and i-carrageenans are in a Kappa-iota hybrid form but one of the two structures may be predominant over the other. The κ-í hybrid state of a structure can be elucidated using specific enzymes, that enrich or diminish the content of one of the two forms. Carrageenans can coexist with their precursors. Carrageenans belonging to different families can coexist in a hybrid structure.

The composition according to the invention preferably comprises one or several lambda carrageenan type sulfated polysaccharides. The sulfated lambda carrageenan type polysaccharide may or may not be chemically modified. Preferably, the sulfated lambda carrageenan type polysaccharide is not chemically modified. Preferably, the molecular weight (PM) of the polysaccharide is between 100,000 and 1,000,000, and more preferably between 250,000 and 800,000. As an example of a lambda carrageenan type polysaccharide, mention may be made of SATIAGUM UTC 10 or SATIAGUM VPC 410 made by DEGUSSA and WELGEENAN ED 1039 made by EUROGUM.

Among branched carboxylated thickening polysaccharides, mention may be made in particular of anionic branched polysaccharides based on glucose, mannose, acetylated mannose, pyruvic acid and glucuronic acid. Such a polysaccharide is xanthan.

Xanthan is a heteropolysaccharide produced on the industrial scale by aerobic fermentation of the *Xanthomonas campestris* bacteria. Its structure is composed of a principal chain of β-D-glucoses bonded in β(1,4), similar to cellulose. One glucose molecule out of two carries a lateral trisaccharidic chain composed of an α-D-mannose, a β-D-glucuronic acid and a terminal β-D-mannose. The internal residue of mannose is generally acetylated on carbon 6. About 30% of terminal mannose residues carry a pyruvate group bonded in chelated form between carbons 4 and 6. The charged glucuronic acids and pyruvic acids can be ionized, and are therefore responsible for the anionic nature of xanthan (negative charge up to pH equal to 1). The content of pyruvate and acetate residues varies depending on the strain of bacteria, the fermentation process, conditions after fermentation and purification steps. These groups can be neutralized in commercial products with Na₊, K₊ or Ca2₊ ions (SATIA, 1986). The neutralized form can be converted into acid form by ion exchange or by dialysis of an acid solution.

Xanthan gums have a molecular weight between 1,000,000 g/mol and 50,000,000 and a viscosity between 0.6 and 1.65 Pa.s for an aqueous composition containing 1% of xanthan gum (measured at 25°C with a Brookfield LVT type viscometer at 60 rpm).

Xanthan gums are represented for example by the products sold under the names Rhodicare^{®} by RHODIA CHIMIE, under the name SATIAXANE^{TMTM} by Cargill Texturizing Solutions (for the food, cosmetic and pharmaceutical industry), under the name NOVAXAN^{TMTM} by ADM, and under the names Kelzan^{®} and Keltrol^{®} by CP-Kelco.

The concentration of thickening polysaccharides used in the compositions according to this invention varies between 0.01 and 20%, preferably between 0.02 and 10%, preferably between 0.03 and 8%,preferably between 0.05 and 5% and even more preferably between 0.1 and 3% by weight relative to the total weight of the composition.

According to one preferred embodiment, the composition according to the disclosure comprises a thickening agent chosen from among cross-linked homopolymers of acrylic acid, cross-linked copolymers of (meth)acrylic acid and C1-C6 alkyl acrylate, copolymers of acrylamido-2-methyl propane sulfonic acid with one or several non-ionic monomers, cross-linked and preferably neutralized homopolymers of 2-acrylamido 2-methylpropane sulfonic acid, hydroxyethylcelluloses, carrageenans and xanthan gum.

According to one preferred embodiment, the composition according to the disclosure comprises a thickening agent chosen from among cross-linked homopolymers of acrylic acid, cross-linked and preferably neutralized homopolymers of 2-acrylamido 2-methylpropane sulfonic acid, hydroxyethylcelluloses, carrageenans and xanthan gum.

The composition according to the invention may also comprise a mixture of several thickening polymers, typically at least two or more thickening polymers. The composition may typically comprise at least one synthetic anionic thickening polymer and at least one thickening agent chosen from among non-ionic thickening polysaccharides, sulfated thickening polysaccharides, branched carboxylated thickening polysaccharides and mixtures thereof.

Preferably, the total concentration of thickening agent(s) used in the compositions according to this invention varies between 0.01 and 50%, preferably between 0.1 and 20%, preferably between 0.2 and 10%, preferably between 0.3 and 8% and even more preferably between 0.5 and 3% by weight of the total weight of the composition.

### Aqueous phase

The composition according to the invention comprises a physiologically acceptable aqueous medium.

Preferably, the composition according to the invention comprises an aqueous medium comprising at least water.

The aqueous medium can comprise at least one other organic solvent soluble in water, at 25°C, chosen for example from among:
- C1-C4 monoalkanols. The term "C₁-C₄ monoalkanol" means any saturated, linear or branched alkane compound with 1 to 4 carbon atoms and a single hydroxyl (OH) function. Monoalkanols in C₁-C₄ present in compositions according to the invention can be chosen from among methanol, ethanol, propanol, isopropanol, butanol or mixtures thereof. In particular, the choice will be ethanol;
- polyols with 2 to 20 carbon atoms, preferably 2 to 6 carbon atoms, such as glycerol, diglycerol, propyleneglycol, isoprene glycol, dipropyleneglycol, butylene glycol, hexylene glycol, 1,3-propanediol, pentylene glycol, simple sugars, hydrosoluble polyalkyleneglycols; and
- mixtures thereof.

Preferably, the composition according to the invention comprises at least one C1-C4 monoalkanol, and more particularly ethanol.

Monoalkanols are generally present in concentrations ranging from 0.2 to 90% by weight, more preferably from 0.5 to 50%, and preferably from 1% to 10% by weight, with respect to the total weight of the composition.

The composition according to the invention comprises at least one polyol, particularly as defined hereinabove. In particular, the presence of at least one polyol can improve cosmetic properties such as slip during application and reduces stickiness. It comprises at least 5% by weight of polyol(s) relative to the total weight of the composition, preferably from 10% to 50% by weight, preferably from 15% to 40% by weight, preferably from 18% to 35% and even more preferably from 20% to 30% by weight.

Preferably, the composition according to the invention comprises a polyol, preferably glycerine, butylene glycol, propylene glycol or dipropylene glycol and mixtures thereof.

The composition comprises preferably 20 to 99% by weight of water relative to the total weight of the composition, preferably from 30 to 90% by weight, preferably from 35 to 80% and preferably from 40 to 70% by weight.

### Acid and/or base

According to one embodiment, the cosmetic composition according to the invention can comprise an acid and/or a base.

Preferably, the composition according to the invention has a pH of between 4 and 10, preferably between 6 and 9, and preferentially between 6.5 and 8.

### Viscosity

The viscosity of compositions according to the invention is preferably at least 0.10 Poises (Pa.s), measured at 25°C with a Rheomat RM100^{®} viscosity meter made by Lamy Rheology. Preferably, it is between 0.20 Poises and 230 Poises, preferably between 0.20 and 15 Poises, preferably between 0.90 and 15 Poises (Pa.s).

According to one particular embodiment, the composition according to the invention further comprises cosmetically acceptable agents and/or excipients.

The term "cosmetically acceptable" means compatible with the skin and/or integuments thereof, having a pleasant color, odor and texture and not giving rise to unacceptable discomfort (tingling, tightness, redness), liable to dissuade the consumer from using the composition.

Compositions according to the invention can be in the form of an aqueous solution, an aqueous gel, but also in the form of an emulsion, particularly an oil-in-water emulsion.

When the composition according to the invention is in an oil-in-water emulsion form, it comprises an oily phase dispersed in an aqueous phase. In this case, the aqueous phase comprises hydrosoluble filters and the specific thickening agent according to the invention. The term "oily phase" as used for the invention refers to a phase that is liquid at 20-25°C and at a pressure of 1.01325 10⁵ Pa. Such an "oily phase" generally comprises at least one oil. The term "oil" denotes any fatty substance that is in liquid form at ambient temperature (20 to 25°C) and at atmospheric pressure (760 mm of Hg). The "fatty substance" comprises at least one "fatty" hydrocarbon chain, in other words a linear hydrocarbon chain with at least 4 carbon atoms, unsaturated or not unsaturated, possibly substituted, and in particular a linear C5-C30 hydrocarbon chain. The oily phase may comprise an oil and/or a liposoluble UV filter.

Preferably, the composition according to the invention does not comprise an oily phase. Preferably, the composition according to the invention comprises less than 5% by weight of an oily phase, in weight of the total weight of the composition, preferably less than 3% by weight, preferably less than 1% by weight. Preferably, compositions according to the invention are oil-free. For the purposes of the invention, the term "oil free" denotes a composition comprising a single liquid phase that is an aqueous phase (a liquid phase containing water). The term "oil" does not cover, for example, hydrosoluble active ingredients, UV filters soluble in water and glycols soluble in water.

The composition according to the invention comprises less than 3% by weight of oil soluble UV filters, in weight of the total weight of the composition, preferably less than 1% by weight. Preferably, compositions according to the invention do not comprise any oil soluble UV filter.
Preferably, the compositions according to the invention are in the form of an aqueous gel, and comprise, preferably consist in, a physiologically acceptable aqueous medium, at least one hydrosoluble filter capable of absorbing UV from 320 to 400 nm (UVA); at least one hydrosoluble filter capable of absorbing UV from 280 to 320 nm (UVB); and at least one thickening agent chosen from among synthetic anionic thickening polymers, non-ionic thickening polysaccharides, sulfated thickening polysaccharides and branched carboxylated thickening polysaccharides; and optionally an acid and/or a base.

Finally, another purpose of this invention is a non-therapeutic method of cosmetic treatment of keratin fibers, preferably the skin, comprising application of a composition according to the invention on said keratin fibers. Such a method is intended particularly to protect keratin fibers, and particularly the skin, against UV radiation.

We will now give concrete examples illustrating the invention, but that are in no way restrictive.

In the examples, the temperature is ambient temperature (20°C) expressed in degrees Celsius unless mentioned otherwise, and the pressure is atmospheric pressure, unless mentioned otherwise.

In the examples, quantities of the ingredients of the compositions are given as a % by weight relative to the total weight of the composition.

### Example 1: preparation and evaluation of comparative compositions

Compositions A to N in Tables 1 and 2 below were prepared as follows.

Compositions A, D, E, F, H, I, J, L, M and N are comparative compositions.

Other comparative compositions are indicated by a star: these are compositions B, C, G et K. Comparative compositions are different by the nature of the thickening agent:
Comparative compositions B and G comprise clay and mica respectively, that are not polymeric.
Comparative composition C comprises phosphated starch.
Comparative composition K comprises xanthan gum. Gellan is a linear polymer (therefore not branched) of partially acylated glucose, rhamnose and glucuronic acid.
In the following tables, the expression "m.a." means "active material".

### Procedure:

1) put the preservatives and water in solution (phase A1);
2) in a second beaker, make the "aqueous filter phase" mixture (phase B): mix water with the filters, TEA and tromethamine;
3) pour phase B into phase A1;
2) put the carbomer into solution and neutralize it;
4) in sequence, add xanthan gum until it is solubilized, and then carrageenan until it is solubilized;
6) when the polyol(s) is (are) present, add it or them and mix at ambient temperature.

**[Table 1]**

| Phase | **INCI** | A | B* | C* | D | E | F | G* |
|---|---|---|---|---|---|---|---|---|
| A1 | Water | Qs 100 | | | | | | |
| | Preservatives | Qs | Qs | Qs | Qs | Qs | Qs | Qs |
| A2 | Clay (FRAMETIME LTX by EPHYLA) | - | 1.8 | - | - | - | - | - |
| | ACRYLAMIDE/SODIUM ACRYLOYLDIMETHYLTAURATE COPOLYMER (and) ISOHEXADECANE (and) POLYSORBATE 80 (Simulgel 600 by Seppic) | - | - | - | - | 3 | - | - |
| | Mica (SUBMICA E by Sensient) | - | - | - | - | - | - | 8 |
| | HYDROXYPROPYL STARCH PHOSPHATE (starch) (Structure XL by Akzo Nobel) | - | - | 2.5 | - | - | - | - |
| | HYDROXYETHYLCELLULOSE | 1 | | - | | | | - |
| | COPOLYMER ACRYLATES (Carbopol Aqua SF1; 30% of m.a.) | - | - | - | - | - | 3 (0.9% m.a.) | - |
| | Triethanolamine (TEA) | - | - | - | 0.5 | - | 0.37 | - |
| | 90% sodium polyacrylate in water (Cosmedia SP) | - | - | - | 2 (1.8% m.a.) | - | - | - |
| B | Water | 10 | 10 | 10 | 10 | 10 | 10 | 10 |
| | Phenylbenzimidazole Sulfonic Acid (Eusolex 232 by Merck) | 8 | 8 | 8 | 8 | 8 | 8 | 8 |
| | Terephthalylidene Dicamphor Sulfonic Acid (Mexoryl SX by Chimex; 33% in m.a.) | 10 (3.3% m.a.) | 10 (3.3% m.a.) | 10 (3.3% m.a.) | 10 (3.3% m.a.) | 10 (3.3% m.a.) | 10 (3.3% m.a.) | 10 (3.3% m.a.) |
| | TEA | 2.5 | 2.5 | 2.5 | 2.5 | 2.5 | 2.5 | 2.5 |
| | Tromethamine | 3.5 | 3.5 | 3.5 | 3.5 | 3.5 | 3.5 | 3.5 |

**[Table 2]**

| **Phas e** | **INCI** | **H** | **I** | **J** | **K*** | **L** | **M** | **N** |
|---|---|---|---|---|---|---|---|---|
| A1 | Water | Qs 100 | | | | | | |
| | Preservatives | Qs | Qs | Qs | Qs | Qs | Qs | Qs |
| A2 | AMMONIUM POLYACRYLOYLDIMETHYL TAURATE (Hostacerin by Clariant) | - | - | - | - | - | - | **1** |
| | AMMONIUM ACRYLOYLDIMETHYLTAURATE/V P COPOLYMER (Aristoflex AVC by Clariant) | 1 | - | - | - | - | - | - |
| | AMMONIUM ACRYLOYLDIMETHYLTAURATE/ STEARETH-25 METHACRYLATE CROSSPOLYMER (Aristoflex HMS by Clariant) | - | **1** | **-** | **-** | - | - | - |
| | CARBOMER (Carbopol 980) | - | **-** | **1.5** | **-** | - | - | - |
| | Gellan gum (KELCOGEL CG LA CP KELCO) | - | **-** | **-** | **1** | - | - | - |
| | Xanthan gum (KELTROL CG-T CP KELCO) | - | - | - | - | **0.5** | **-** | - |
| | CARRAGEENAN (SATIAGUM VPC 410 CARGILL) | - | | - | - | **-** | **1** | - |
| | Triethanolamine (TEA) | - | - | 1.88 | - | **-** | **-** | - |
| B | Water | 10 | 10 | 15 | 15 | 15 | 15 | 15 |
| | Phenylbenzimidazole Sulfonic Acid (Eusolex 232 by Merck) | 8 | 8 | 8 | 8 | 8 | 8 | 8 |
| | Terephthalylidene Dicamphor Sulfonic Acid (Mexoryl SX by Chimex; 33% in m.a.) | 10 (3.3 % m.a.) | 10 (3.3 % m.a.) | 10 (3.3 % m.a.) | 10 (3.3 % m.a.) | 10 (3.3 % m.a.) | 10 (3.3 % m.a.) | 10 (3.3 % m.a.) |
| | TEA | 2.5 | 2.5 | 2.5 | 2.5 | 2.5 | 2.5 | 2.5 |
| | Tromethamine | 3.5 | 3.5 | 3.5 | 3.5 | 3.5 | 3.5 | 3.5 |

Once prepared, the different compositions are tested for their appearance (homogeneity/compatibility), and their viscosity and turbidity are measured.

The viscosity and turbidity are measured as described in the description.

The results are given in Tables 3 and 4.

**[Table 3]**

| **Formula** | **A** | **B*** | **C*** | **D** | **E** | **F** | **G*** |
|---|---|---|---|---|---|---|---|
| Homoge neity | Homogen eous | **Non homogen eous (non-smooth appearan ce)** | **Non homogen eous (phase change)** | Homogen eous | Homogen eous | Homogen eous | **Non homogen eous (phase change)** |
| Viscosity (in Poises) | 3.12 | - | - | <0.2 | <0.2 | <0.2 | - |
| Turbidity (in NTU) | 3.45 | - | - | Not measurabl e | Not measurabl e | 5.5 | - |

**[Table 4]**

| **Formula** | **H** | **I** | **J** | **K*** | **L** | **M** | **N** |
|---|---|---|---|---|---|---|---|
| Homogen eity | Homogen eous | Homogen eous | Homogen eous | **Not homogen eous (appearan ce not smooth)** | Homogen eous | Homogen eous | Homogen eous |
| Viscosity (in Poises) | <0.2 | <0.2 | 13.2 | **-** | 0.93 | 1.50 | 0.29 |
| Turbidity (in NTU) | 36 | 139 | 140 | - | 16 | 14 | 20.5 |

The results show that the comparative compositions B, C, G and K have a changed phase appearance, and are therefore not homogeneous.

On the contrary, the other comparative compositions are homogeneous.

### Example 2: preparation and evaluation of compositions according to the invention

Comparative composition O, and compositions P and Q according to the invention, comprising a polyol (see Table 5), are prepared as described in example 1.

Their homogeneity and transparency, and their stickiness and slip during application, are evaluated.

**[Table 5]**

| Phase | **INCI** | **O** (comparative) | P | Q |
|---|---|---|---|---|
| A1 | Water | Qs 100 | | |
| | Preservatives | Qs | Qs | Qs |
| A2 | Xanthan gum (KELTROL CG-T CP KELCO) | 1 | 1 | 1 |
| B | Water | 15 | 15 | 15 |
| | Phenylbenzimidazole Sulfonic Acid (Eusolex 232 by Merck) | 8 | 8 | 8 |
| | Terephthalylidene Dicamphor Sulfonic Acid (Mexoryl SX by Chimex; 33% in m.a.) | 10 (3.3% m.a.) | 10 (3.3% m.a.) | 10 (3.3% m.a.) |
| | TEA | 2.5 | 2.5 | 2.5 |
| | Tromethamine | 3.5 | 3.5 | 3.5 |
| C | Butylene glycol | | 20 | |
| | Propylene glycol | | | 20 |
| Homogeneity | | Homogeneous | Homogeneous | Homogeneous |

Compositions O (comparative), and P and Q according to the invention are homogeneous and transparent.

Furthermore, the presence of glycol(s) as in compositions P and Q, reduces stickiness and improves slip during application.

### Example 3: preparation and evaluation of compositions according to the invention

Comparative composition R and composition S according to the invention (see Table 6), are prepared as described in example 1.

Their homogeneity and transparency, and their stickiness and slip during application, are evaluated.

**[Table 6]**

| | | R (comparative) | **S** |
|---|---|---|---|
| Gel phase | Water | Qs 100 | |
| | Preservatives | QS | |
| | **CARBOMER** (carbopol 980) | 0.5 | 0.5 |
| | Triethanolamine (TEA) | 0.7 | 0.7 |
| | **Xanthan gum** (KELTROL CG-T CP KELCO) | 0.3 | 0.3 |
| | **CARRAGEENAN** (SATIAGUM VPC 410 CARGILL) | 0.5 | **0.5** |
| Aqueous filter phase | Water | 15 | 15 |
| | Phenylbenzimidazole Sulfonic Acid (Eusolex 232 by Merck) | 8 | 8 |
| | Terephthalylidene Dicamphor Sulfonic Acid (Mexoryl SX by Chimex; 33% in m.a.) | 10 (3.3% m.a.) | 10 (3.3% m.a.) |
| | TEA | 2.5 | 2.5 |
| | Tromethamine | 3.5 | 3.5 |
| | **Glycerin** | - | **2** |
| | **Propylene glycol** | - | **19** |
| | **Butylene glycol** | - | **6.5** |
| Homogeneity | | Homogeneous | Homogeneous |
| Turbidity (in NTU) | | 71 | 50 |

The compositions are homogeneous and transparent.

Furthermore, the presence of polyols as in composition S, reduces stickiness and improves slip during application.

## Claims

1. Composition, particularly a cosmetic composition, comprising the following in a physiologically acceptable aqueous medium:
between 0.2% and 40% by weight relative to the total weight of composition of at least one hydrosoluble filter capable of absorbing UV from 320 to 400 nm (UVA) chosen from terephthalylidene dicamphor sulfonic acid and the Disodium Phenyl Dibenzimidazole Tetra-sulfonate compound, and
between 0.2% and 40% by weight relative to the total weight of composition of at least one hydrosoluble filter capable of absorbing UV from 280 to 320 nm (UVB) that is Phenylbenzimidazole Sulfonic acid; and
at least one thickening agent chosen from among synthetic anionic thickening polymers, non-ionic thickening polysaccharides, sulfated thickening polysaccharides and branched carboxylated thickening polysaccharides,
wherein the synthetic anionic thickening polymers are chosen from cross-linked homopolymers of acrylic acid; cross-linked copolymers of (meth)acrylic acid and C1-C6 alkyl acrylate; and copolymers of acrylamido-2-methyl propane sulfonic acid or its salts and of one or several non-ionic monomers,
wherein the non-ionic thickening polysaccharides are chosen from hydroxyalkylcelluloses,
wherein the sulfated thickening polysaccharides are chosen from carrageenans,
wherein the branched carboxylated thickening polysaccharide is xanthan gum,
wherein the composition comprises less than 3% by weight of oil soluble UV filters in weight of the total weight of the composition,
wherein the composition comprises at least one polyol having 2 to 20 carbon atoms in a quantity at least 5% by weight relative to the total weight of the composition.

2. Composition according to claim 1, **characterized in that** the hydrosoluble filter capable of absorbing UVA is terephthalylidene dicamphor sulfonic acid.

3. Composition according to claim 1 or 2, **characterized in that** it comprises a total quantity of hydrosoluble filters equal to between 0.2% and 40% by weight, preferably between 0.5% and 40% by weight relative to the total weight of composition, preferably between 1% and 30% by weight, and preferably between 3% and 20% by weight.

4. Composition according to one of claims 1 to 3, **characterized in that** it comprises at least one C1-C4 mono-alkanol, preferably chosen from among methanol, ethanol, propanol, isopropanol, butanol and mixtures thereof; and/or at least one polyol chosen from among glycerol, diglycerol, propyleneglycol, isoprene glycol, dipropyleneglycol, butylene glycol, hexylene glycol, 1,3-propanediol, pentylene glycol, simple sugars, hydrosoluble polyalkyleneglycols and mixtures thereof.

5. Composition according to claim 4, **characterized in that** it comprises at least one C1-C4 mono-alkanol, in a quantity varying from 0.2 to 90% by weight, preferably 0.5 to 50%, and preferably 1% to 10% by weight relative to the total weight of the composition; and/or the at least one polyol in a quantity from 10% to 50% by weight, preferably 15% to 40% by weight, preferably 18 to 35%, and even more preferably from 20% to 30% by weight.

6. Composition according to one of claims 1 to 5, **characterized in that** the copolymers of acrylamido-2-methyl propane sulfonic acid or its salts and of one or several non-ionic monomers are cross-linked.

7. Composition according to one of claims 1 to 6, **characterized in that** the composition is transparent and presents a turbidity value of less than 800 NTU, preferably less than 500 NTU, preferably less than 200 NTU, preferably less than 150 NTU, and preferably less than 100 NTU, wherein the turbidity measurement is made with a model 2100P turbidity meter made by the Hach Company, the tubes used for the measurement being references AR397A cat 24347-06, and the measurements are made at ambient temperature.

8. Composition according to one of claims 1 to 7, **characterized in that** it is in the form of an aqueous gel, and comprises, preferably consists in, a physiologically acceptable aqueous medium, at least one hydrosoluble filter capable of absorbing UV from 320 to 400 nm (UVA) chosen from terephthalylidene dicamphor sulfonic acid and the Disodium Phenyl Dibenzimidazole Tetra-sulfonate compound; at least one hydrosoluble filter capable of absorbing UV from 280 to 320 nm (UVB) that is Phenylbenzimidazole Sulfonic acid; and at least one thickening agent chosen from among synthetic anionic thickening polymers, non-ionic thickening polysaccharides, sulfated thickening polysaccharides and branched carboxylated thickening polysaccharides;
wherein the synthetic anionic thickening polymers are chosen from cross-linked homopolymers of acrylic acid; cross-linked copolymers of (meth)acrylic acid and C1-C6 alkyl acrylate; and copolymers of acrylamido-2-methyl propane sulfonic acid or its salts and of one or several non-ionic monomers,
wherein the non-ionic thickening polysaccharides are chosen from hydroxyalkylcelluloses, wherein the sulfated thickening polysaccharides are chosen from carrageenans,
wherein the branched carboxylated thickening polysaccharide is xanthan gum,
and optionally an acid and/or a base.

9. Composition according to claim 6, **characterized in that** the copolymer of acrylamido-2-methyl propane sulfonic acid or its salts and of one or several non-ionic monomers is chosen from among copolymers of acrylamido-2-methyl propane sulfonic acid and vinylpyrrolidone, cross-linked copolymers of acrylamide/acrylamido-2-methyl propane sodium sulfonate, copolymers of acrylamido-2-methyl propane sulfonic acid and hydroxyethyl acrylate and hydrophobically modified copolymers of acrylamido-2-methyl propane sulfonic acid.

10. Composition according to one of claims 1 to 9, **characterized in that** the synthetic anionic thickening polymer is present as active material in a quantity varying from 0.05 to 35% by weight relative to the total weight of the composition, and preferably from 0.1 to 20% by weight, and even more particularly from 0.2 to 10% by weight, and preferably from 0.25 to 5% by weight, and even more particularly from 0.3 to 3% by weight.

11. Composition according to one of claims 1 to 5, **characterized in that** the non-ionic thickening polysaccharide is chosen from among hydroxyethylcelluloses and hydroxypropylcelluloses.

12. Composition according to one of claims 1 to 5, **characterized in that** the sulfated thickening polysaccharide is a lambda carrageenan type sulfated polysaccharide.

13. Composition according to one of claims 1 to 12, **characterized in that** the thickening polysaccharide is present in a quantity varying between 0.01 and 20%, preferably between 0.02 and 10%, preferably between 0.03 and 8%, preferably between 0.05 and 5% and even more preferably between 0.1 and 3% by weight relative to the total weight of the composition.

14. Composition according to one of claims 1 to 13, **characterized in that** it comprises less than 5% by weight of an oily phase, in weight of the total weight of the composition, preferably less than 3% by weight, preferably less than 1% by weight, preferably is oil-free; and/or it comprises less than 1% by weight of oil soluble UV filters, in weight of the total weight of the composition.

## Patentansprüche

1. Zusammensetzung, insbesondere kosmetische Zusammensetzung,
umfassend das Folgende in einem physiologisch annehmbaren wässrigen Medium:
zwischen 0,2 und 40 Gewichts-%, bezogen auf das Gesamtgewicht der Zusammensetzung, mindestens eines wasserlöslichen Filters, der in der Lage ist, UV-Strahlung von 320 bis 400 nm (UVA), ausgewählt aus Terephthalylidendicamphersulfonsäure und der Verbindung Dinatriumphenyldibenzimidazoltetrasulfonat, zu absorbieren, und
zwischen 0,2 und 40 Gewichts-%, bezogen auf das Gesamtgewicht der Zusammensetzung, mindestens eines wasserlöslichen Filters, der in der Lage ist, UV-Strahlung von 280 bis 320 nm (UVB) zu absorbieren, der Phenylbenzimidazolsulfonsäure ist; und
mindestens ein Verdickungsmittel, das ausgewählt ist aus synthetischen anionischen verdickenden Polymeren, nichtionischen verdickenden Polysacchariden, sulfatierten verdickenden Polysacchariden und verzweigten carboxylierten verdickenden Polysacchariden,
wobei die synthetischen anionischen Verdickungspolymere ausgewählt sind aus vernetzten Homopolymeren von Acrylsäure; vernetzten Copolymeren von (Meth)acrylsäure und C1-C6-Alkylacrylat; und Copolymeren von Acrylamido-2-methylpropansulfonsäure oder ihren Salzen und einem oder mehreren nichtionischen Monomeren,
wobei die nichtionischen verdickenden Polysaccharide ausgewählt sind aus Hydroxyalkylcellulosen,
wobei die sulfatierten verdickenden Polysaccharide ausgewählt sind aus Carrageenanen,
wobei das verzweigte carboxylierte verdickende Polysaccharid Xanthangummi ist,
wobei die Zusammensetzung weniger als 3 Gewichts-% öllöslicher UV-Filter, bezogen auf das Gesamtgewicht der Zusammensetzung, umfasst,
wobei die Zusammensetzung mindestens ein Polyol umfasst, das 2 bis 20 Kohlenstoffatome in einer Menge von mindestens 5 Gewichts-%, bezogen auf das Gesamtgewicht der Zusammensetzung, aufweist.

2. Zusammensetzung nach Anspruch 1, **dadurch gekennzeichnet, dass** der wasserlösliche Filter, der in der Lage ist, UVA zu absorbieren, Terephthalylidendicamphersulfonsäure ist.

3. Zusammensetzung nach Anspruch 1 oder 2, **dadurch gekennzeichnet, dass** sie eine Gesamtmenge an wasserlöslichen Filtern umfasst, die zwischen 0,2 und 40 Gewichts-% ist, vorzugsweise zwischen 0,5 und 40 Gewichts-%, bezogen auf das Gesamtgewicht der Zusammensetzung, vorzugsweise zwischen 1 und 30 Gewichts-% und vorzugsweise zwischen 3 und 20 Gewichts-%.

4. Zusammensetzung nach einem der Ansprüche 1 bis 3, **dadurch gekennzeichnet, dass** sie mindestens ein C1-C4-Monoalkanol, vorzugsweise ausgewählt aus Methanol, Ethanol, Propanol, Isopropanol, Butanol und deren Gemischen, und/oder mindestens ein Polyol, ausgewählt aus Glycerol, Diglycerol, Propylenglykol, Isopren-glykol, Dipropylenglykol, Butylenglykol, Hexylenglykol, 1,3-Propandiol, Pentylenglykol, einfachen Zuckern, wasserlöslichen Polyalkylenglykolen und deren Gemischen, umfasst.

5. Zusammensetzung nach Anspruch 4, **dadurch gekennzeichnet, dass** sie mindestens ein C1-C4-Monoalkanol in einer Menge, die von 0,2 bis 90 Gewichts-%, vorzugsweise 0,5 bis 50 Gewichts-% und vorzugsweise 1 bis 10 Gewichts-%, bezogen auf das Gesamtgewicht der Zusammensetzung, variiert und/oder mindestens ein Polyol in einer Menge von 10 bis 50 Gewichts-%, vorzugsweise 15 bis 40 Gewichts-%, vorzugsweise 18 bis 35 Gewichts-% und bevorzugter 20 bis 30 Gewichts-% umfasst.

6. Zusammensetzung nach einem der Ansprüche 1 bis 5, **dadurch gekennzeichnet, dass** die Copolymere von Acrylamido-2-methylpropansulfonsäure oder deren Salzen und einem oder mehreren nichtionischen Monomeren vernetzt sind.

7. Zusammensetzung nach einem der Ansprüche 1 bis 6, **dadurch gekennzeichnet, dass** die Zusammensetzung transparent ist und einen Trübungswert von weniger als 800 NTU, vorzugsweise weniger als 500 NTU, vorzugsweise weniger als 200 NTU, vorzugsweise weniger als 150 NTU und vorzugsweise weniger als 100 NTU aufweist, wobei die Trübungsmessung mit einem Trübungsmessgerät Modell 2100P der Firma Hach an Referenzröhrchen AR397A (cat. 24347-06) bei Umgebungstemperatur vorgenommen wird.

8. Zusammensetzung nach einem der Ansprüche 1 bis 7, **dadurch gekennzeichnet, dass** sie in Form eines wässrigen Gels ist und ein physiologisch annehmbares wässriges Medium, mindestens einen wasserlöslichen Filter, der in der Lage ist, UV-Strahlung von 320 bis 400 nm (UVA) zu absorbieren, ausgewählt aus Terephthalylidendicamphersulfonsäure und der Verbindung Dinatriumphenylbenzimidazoltetrasulfonat, umfasst, vorzugsweise daraus besteht; mindestens einen wasserlöslichen Filter, der in der Lage ist, UV-Strahlen von 280 bis 320 nm (UVB) zu absorbieren und der Phenylbenzimidazolsulfonsäure ist; und mindestens ein Verdickungsmittel, das ausgewählt ist aus synthetischen anionischen verdickenden Polymeren, nichtionischen verdickenden Polysacchariden, sulfatierten verdickenden Polysacchariden und verzweigten carboxylierten verdickenden Polysacchariden;
wobei die synthetischen anionischen Verdickungspolymere ausgewählt sind aus vernetzten Homopolymeren von Acrylsäure; vernetzten Copolymeren von (Meth)acrylsäure und C1-C6-Alkylacrylat; und Copolymeren von Acrylamido-2-methylpropansulfonsäure oder ihren Salzen und einem oder mehreren nichtionischen Monomeren,
wobei die nichtionischen verdickenden Polysaccharide ausgewählt sind aus Hydroxyalkylcellulosen,
wobei die sulfatierten verdickenden Polysaccharide ausgewählt sind aus Carrageenanen, wobei das verzweigte, carboxylierte, verdickende Polysaccharid Xanthangummi ist,
und optional eine Säure und/oder eine Base.

9. Zusammensetzung nach Anspruch 6, **dadurch gekennzeichnet, dass** das Copolymer von Acrylamido-2-methylpropansulfonsäure oder dessen Salze und ein oder mehrere nichtionische Monomere ausgewählt ist aus: Copolymeren von Acrylamido-2-methylpropansulfonsäure und Vinylpyrrolidon, vernetzten Copolymeren von Acrylamid/Acrylamido-2-methylpropan-Natriumsulfonat, Copolymeren von Acrylamido-2-methylpropansulfonsäure und Hydroxyethylacrylat sowie hydrophob modifizierten Copolymeren von Acrylamido-2-methylpropansulfonsäure.

10. Zusammensetzung nach einem der Ansprüche 1 bis 9, **dadurch gekennzeichnet, dass** das synthetische anionische Verdickungspolymer als aktives Material in einer Menge von 0,05 bis 35 Gewichts-%, bezogen auf das Gesamtgewicht der Zusammensetzung, und vorzugsweise von 0,1 bis 20 Gewichts- %, und noch spezieller von 0,2 bis 10 Gewichts-%, und vorzugsweise von 0,25 bis 5 Gewichts-%, und noch spezieller von 0,3 bis 3 Gewichts-% vorhanden ist.

11. Zusammensetzung nach einem der Ansprüche 1 bis 5, **dadurch gekennzeichnet, dass** das nichtionische verdickende Polysaccharid ausgewählt ist aus Hydroxyethylcellulosen und Hydroxypropylcellulosen.

12. Zusammensetzung nach einem der Ansprüche 1 bis 5, **dadurch gekennzeichnet, dass** das sulfatierte, verdickende Polysaccharid vom Typ Lambda-Carrageenan ist.

13. Zusammensetzung nach einem der Ansprüche 1 bis 12, **dadurch gekennzeichnet, dass** das verdickende Polysaccharid in einer Menge vorhanden ist, die zwischen 0,01 und 20 %, vorzugsweise zwischen 0,02 und 10 %, vorzugsweise zwischen 0,03 und 8 %, vorzugsweise zwischen 0,05 und 5 % und bevorzugter zwischen 0,1 und 3 Gewichts-%, bezogen auf das Gesamtgewicht der Zusammensetzung, variiert.

14. Zusammensetzung nach einem der Ansprüche 1 bis 13, **dadurch gekennzeichnet, dass** sie weniger als 5 Gewichts-% einer öligen Phase, bezogen auf das Gesamtgewicht der Zusammensetzung, umfasst, vorzugsweise weniger als 3 Gewichts-%, vorzugsweise weniger als 1 Gewichts-%, vorzugsweise ölfrei ist; und/oder dass sie weniger als 1 Gewichts-% öllösliche UV-Filter, bezogen auf das Gesamtgewicht der Zusammensetzung, umfasst.

## Revendications

1. Composition, en particulier composition cosmétique, comprenant, dans
un milieu aqueux physiologiquement acceptable :
entre 0,2% et 40% en poids par rapport au poids total de composition d'au moins un filtre hydrosoluble capable d'absorber les UV de 320 à 400 nm (UVA) choisi parmi l'acide terephthalylidène dicamphre sulfonique et le composé Disodium Phenyl Dibenzimidazole Tetra-sulfonate, et
entre 0,2% et 40% en poids par rapport au poids total de composition d'au moins un filtre hydrosoluble capable d'absorber les UV de 280 à 320 nm (UVB) qui est l'acide Phenylbenzimidazole Sulfonique ; et
au moins un agent épaississant choisi parmi les polymères épaississants synthétiques anioniques, les polysaccharides épaississants non ioniques, les polysaccharides épaississants sulfatés et les polysaccharides épaississants ramifiés carboxylés,
dans laquelle les polymères épaississants synthétiques anioniques sont choisis parmi les homopolymères réticulés d'acide acrylique, les copolymères réticulés d'acide (méth)acrylique et d'acrylate d'alkyle en C1-C6 ; et les copolymères d'acide acrylamido-2-méthyl propane sulfonique ou ses sels et d'un ou plusieurs monomères non ioniques,
dans laquelle les polysaccharides épaississants non ioniques sont choisis parmi les hydroxyalkylcelluloses,
dans laquelle les polysaccharides épaississants sulfatés sont choisis parmi les carraghénanes,
dans laquelle le polysaccharide épaississant ramifié carboxylé est la gomme de xanthane, dans laquelle la composition comprend moins de 3% en poids de filtres UV liposolubles par rapport au poids total de composition,
dans laquelle la composition comprend au moins un polyol ayant de 2 à 20 atomes de carbone en une quantité d'au moins 5% en poids par rapport au poids total de composition.

2. Composition selon la revendication 1, **caractérisée en ce que** le filtre hydrosoluble capable d'absorber les UVA est l'acide terephthalylidène dicamphre sulfonique.

3. Composition selon la revendication 1 ou 2, **caractérisée en ce qu'**elle comprend une quantité totale de filtres hydrosolubles comprise entre 0,2% et 40% en poids, de préférence entre 0,5% et 40% en poids par rapport au poids total de composition, de préférence entre 1% et 30% en poids, de préférence entre 3% et 20% en poids.

4. Composition selon l'une des revendications 1 à 3, **caractérisée en ce qu'**elle comprend au moins un mono-alcanol en C1-C4, de préférence choisi parmi le méthanol, l'éthanol, le propanol, l'isopropanol, le butanol et leurs mélanges ; et/ou au moins un polyol choisi parmi le glycérol, le diglycérol, le propylèneglycol, l'isoprène glycol, le dipropylèneglycol, le butylène glycol, l'hexylène glycol, le 1,3-propanediol, le pentylène glycol, les sucres simples, les polyalkylèneglycols hydrosolubles et leurs mélanges.

5. Composition selon la revendication 4, **caractérisée en ce qu'**elle comprend au moins un mono-alcanol en C1-C4 en quantité allant de 0,2 à 90% en poids, plus préférentiellement de 0,5 à 50 %, et de manière préférée de 1% à 10% en poids par rapport au poids total de la composition ; et/ou au moins un polyol en quantité de 10% à 50% en poids, de préférence de 15% à 40% en poids, de préférence de 18% à 35% et encore plus préférentiellement de 20% à 30% en poids.

6. Composition selon l'une des revendications 1 à 5, **caractérisée en ce que** les copolymères d'acide acrylamido-2-méthyl propane sulfonique ou ses sels et d'un ou plusieurs monomères non ioniques sont réticulés.

7. Composition selon l'une des revendications 1 à 6, **caractérisée en ce que** la composition est transparente et présente une valeur de turbidité inférieure à 800 NTU, de préférence inférieure à 500 NTU, de préférence inférieure à 200 NTU, de préférence inférieure à 150 NTU, et de préférence inférieure à 100 NTU, dans laquelle la mesure de turbidité est réalisée sur un modèle de turbidimètre 2100P de Hach Company, les tubes utilisés pour la mesure étant référencés AR397A cat 24347-06, et les mesures sont faites à température ambiante.

8. Composition selon l'une des revendications 1 à 7, **caractérisée en ce qu'**elle est sous forme de gel aqueux, et comprend, de préférence consiste en, un milieu aqueux physiologiquement acceptable, au moins un filtre hydrosoluble capable d'absorber les UV de 320 à 400 nm (UVA) choisi parmi l'acide terephthalylidène dicamphre sulfonique et le composé Disodium Phenyl Dibenzimidazole Tetra-sulfonate ; au moins un filtre hydrosoluble capable d'absorber les UV de 280 à 320 nm (UVB) qui est l'acide Phenylbenzimidazole Sulfonique ; et au moins un agent épaississant choisi parmi les polymères épaississants synthétiques anioniques, les polysaccharides épaississants non ioniques, les polysaccharides épaississants sulfatés et les polysaccharides épaississants ramifiés carboxylés ;
dans laquelle les polymères épaississants synthétiques anioniques sont choisis parmi les homopolymères réticulés d'acide acrylique ; les copolymères réticulés d'acide (méth)acrylique et d'acrylate d'alkyle en C1-C6 ; et les copolymères d'acide acrylamido-2-méthyl propane sulfonique ou ses sels et d'un ou plusieurs monomères non ioniques,
dans laquelle les polysaccharides épaississants non ioniques sont choisis parmi les hydroxyalkylcelluloses,
dans laquelle les polysaccharides épaississants sulfatés sont choisis parmi les carraghénanes,
dans laquelle le polysaccharide épaississant ramifié carboxylé est la gomme de xanthane, et optionnellement un acide et/ou une base.

9. Composition selon la revendication 6, **caractérisée en ce que** le copolymère d'acide acrylamido-2-méthyl propane sulfonique ou ses sels et d'un ou plusieurs monomères non ioniques est choisi parmi les copolymères d'acide acrylamido-2-methyl propane sulfonique et de vinylpyrrolidone, les copolymères réticulés acrylamide/acrylamido-2-methyl propane sulfonate de sodium, les copolymères d'acide acrylamido-2-méthyl propane sulfonique et d'hydroxyéthyl acrylate et les copolymères d'acide acrylamido-2-méthyl propane sulfonique modifiés hydrophobiquement.

10. Composition selon l'une des revendications 1 à 9, **caractérisée en ce que** le polymère épaississant synthétique anionique est présent en matière active en quantité allant de 0,05 à 35% en poids par rapport au poids total de la composition, et de préférence de 0,1 à 20% en poids, et encore plus particulièrement de 0,2 à 10% en poids, de préférence de 0,25 à 5% en poids et encore plus particulièrement de 0,3 à 3% en poids.

11. Composition selon l'une des revendications 1 à 5, **caractérisée en ce que** le polysaccharide épaississant non ionique est choisi parmi des hydroxyéthylcelluloses et des hydroxypropylcelluloses.

12. Composition selon l'une des revendications 1 à 5, **caractérisée en ce que** le polysaccharide épaississant sulfaté est un polysaccharide sulfaté de type carraghénane lambda.

13. Composition selon l'une des revendications 1 à 12, **caractérisée en ce que** le polysaccharide épaississant est présent en quantité allant de 0,01 à 20%, de préférence de 0,02 à 10%, de préférence de 0,03 à 8%, de préférence de 0,05 à 5% et de manière encore plus préférée de 0,1 à 3% en poids par rapport au poids total de la composition.

14. Composition selon l'une des revendications 1 à 13, **caractérisée en ce qu'**elle comprend moins de 5% en poids d'une phase huileuse par rapport au poids total de composition, de préférence moins de 3% en poids, de préférence moins de 1% en poids, de préférence est exempte de phase huileuse ; et/ou elle comprend moins de 1% en poids de filtres UV liposolubles par rapport au poids total de composition.
